## Europäisches Patentamt
## European Patent Office
## Office européen des brevets

(11) Publication number: **0 120 612**
**B1**

(12) **EUROPEAN PATENT SPECIFICATION**

(45) Date of publication of patent specification: **19.04.89**

(51) Int. Cl.⁴: **A 61 F 13/02**

(21) Application number: **84301250.1**

(22) Date of filing: **27.02.84**

(54) Blister pad adhesive bandage.

(30) Priority: **28.02.83 US 470417**

(43) Date of publication of application:
**03.10.84 Bulletin 84/40**

(45) Publication of the grant of the patent:
**19.04.89 Bulletin 89/16**

(84) Designated Contracting States:
**AT BE CH DE IT LI LU NL SE**

(56) References cited:
**EP-A-0 109 280**
**FR-A-2 298 339**
**US-A-3 025 854**
**US-A-3 067 747**
**US-A-3 900 027**
**US-A-4 307 721**

(73) Proprietor: **Johnson & Johnson Products Inc.**
**501 George Street**
**New Brunswick New Jersey 08903 (US)**

(72) ·Inventor: **Lauritzen, Nels Jens**
**34 Orris Avenue**
**Piscataway New Jersey 08854 (US)**

(74) Representative: **Jones, Alan John et al**
**CARPMAELS & RANSFORD 43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to adhesive bandages comprising a central pad area and adjacent adhesive areas, and more particularly, to blister pad adhesive bandages constructed entirely from heat-fusible, nonwoven fabric materials.

Adhesive bandages comprising a central pad area and adjacent adhesive areas are well-known in the art and popular as first aid wound dressings. Current bandages generally comprise an elongated strip of cloth or plastic backing material coated on one surface with a pressure sensitive adhesive. A gauze or sponge pad is secured to the adhesive surface in a central location to serve as the wound cover. The wound facing surface of the pad may be plastic-coated or otherwise treated to prevent the pad from adhering to the wound. Plastic-coated release strips are placed over the adhesive areas and the entire assembly is placed in a sealed package and sterilized to be ready for use.

An alternate form of adhesive bandage, generally referred to as an island bandage, comprises a generally square, round or oval adhesive coated backing material with a centrally located pad forming an island surrounded by the adhesive surface. The adhesive surface is similarly covered by release paper before the bandage is packaged and sterilized.

The adhesive bandages of the prior art are characterized by their construction of two basic components the adhesive coated backing material and the wound covering pad material. The pad material may be dry or impregnated with various bactericides or other wound treatment medicaments. The capacity of the pad to absorb and hold such compositions is a limiting factor on the amount of such material which may be incorporated into the bandages.

US-A-3 900 027 (Keedwell) describes a process for making integral absorbent pad bandages from non-woven thermoplastic fibrous sheet material by compressing the sheet in selected portions to reduce thickness and porosity and delimit an absorbent pad portion having a greater thickness. The resulting sheet has a plurality of juxtaposed integral absorbent pad bandages which can be cut off to obtain individual bandages. The individual bandages are in one piece and can have adhesive applied thereto if desired.

US-A-3 025 854 (Scholl) discloses an adhesive bandage constructed of:

a heat fusible base portion; and

a bulky, heat fusible pad portion centrally located on said base portion;

said pad portion having been provided separate from said base portion and being heat fused to said base portion along its perimeter;

said base portion including adhesive-coated areas extending outwardly from said pad portion.

The Scholl patent also discloses a method for making such bandages.

It is an object of the present invention to provide an improved adhesive bandage. It is a further object of this invention to provide a low cost adhesive bandage through the use of inexpensive materials and low cost manufacturing techniques. A further object of this invention is to provide an adhesive bandage having a high loading of wound treatment medicament in the pad area. These and other objects of the present invention will be apparent from the ensuing description and claims of the invention.

The present invention provides an adhesive bandage which is characterised in that said pad portion is formed of a non-woven fabric and is heat fused to said base portion around its entire perimeter, whereby the interior of said pad portion is totally enclosed and forms a blister pad on said base portion; and

said adhesive coated areas of said base portion have been compacted and heat fused from a bulky non-woven fabric into a dense, sheet-like structure.

The bandage may comprise a strip bandage or an island bandage.

The present invention also provides a method, for the continuous production of a plurality of adhesive bandage strips from a heat-fusible material, comprising the steps of:

a) providing a continuous source of base material having a width corresponding to the desired length of the bandage strip;

b) providing a continuous source of pad material having a width at least equal to the desired length of the pad; centering the pad material on the base material;

c) securing the pad material on the base material by heat fusing the edges of the pad material to the base material;

e) applying an adhesive coating to one surface of the side portions of said base material extending from the pad material;

f) covering said adhesive-coated side portions of said base material with a release liner; and

g) cutting the resulting composite material into narrow strips transversely to the longitudinal machine direction thereof,

characterised in that:

said base material comprises a bulky, non-woven fabric;

said pad material comprises a separate bulky, non-woven fabric;

during said securing step d), the edges of the pad material are compacted;

prior to said adhesive applying step e), said side portions of the base material are compacted and heat fused to obtain a dense, sheet-like structure for receiving the adhesive; and

during the cutting step g), the cut edges of the pad material are heat fused to seal the pad material to the base material, whereby there are formed individual adhesive bandages comprising a central enclosed blister pad portion and adhesive-coated side portions extending therefrom.

The present invention further provides a method, for the continuous production of a plurality of adhesive island bandages from a heat-fusible material, comprising the steps of:

a) providing a continuous source of base material having a width corresponding to the desired width of the bandage;

b) providing a continuous source of pad material having a width at least equal to the desired width of the pad;

c) centering the pad material on the base material;

d) securing the pad material to the base material by heat-fusing the pad material to the base material;

e) applying an adhesive coating to one surface of the heat-fused portions of said pad material and the portions of the base material extending beyond the pad area;

f) covering said adhesive-coated portions of said material with a release liner; and

g) cutting bandages from the resulting composite material

characterised in that:

said base material comprises a bulky, non-woven fabric;

said pad material comprises a separate bulky, non-woven fabric;

during said securing step d), the pad material is heat-fused to the base material all around the pad material perimeter and the perimeter of the pad material is compacted;

prior to said adhesive applying step e), the portions of the base material extending beyond the pad area are compacted and heat fused to obtain a dense, sheet-like structure for receiving the adhesive; and

during the cutting step g), there are formed individual adhesive island bandages comprising a central enclosed blister pad portion surrounded by adhesive-coated side portions extending therefrom.

In accordance with the present invention, adhesive bandages comprising an elongated strip of material having a centrally-located blister pad and adjacent adhesive portions extending from each side of the pad area are prepared from a heat-bondable, absorbent, non-woven fabric material. The blister pad is applied to the center of the bandage strip and secured by heat bonding. A medicated gel or other material may be deposited on the bandage strip and covered with the blister pad to provide a medicated bandage. The portions of the bandage extending beyond the pad area are permanently compacted to provide a surface suitable for coating with adhesive. The pad area is preferably left uncompacted to retain loft and absorbency, but may be heat glazed to provide a nonadhering wound release surface.

Strip bandages are conveniently prepared according to the present invention from a first, continuous base-fabric having a width equal to the overall length of the desired bandage, and a second, continuous pad-fabric having a width at least equal to the desired pad area. The base fabric is fed through a station where a medicated gel or other material is deposited onto the center of the moving fabric if desired. The pad fabric is then applied as a blister dome over the deposited material and the combined fabrics passed through a hot roll calendering station where the edges of the pad and the portions of the base fabric extending from each side of the pad area are compacted and heat-fused to secure the pad and to form a dense, nonwoven sheet-like structure extending from the pad area.

The composite fabric is next passed through an adhesive application station where a pressure-sensitive adhesive, preferably a hot melt-type adhesive, is coated onto one surface of the compacted base fabric on either side of the pad area. Adhesive release liners are applied over the adhesive area and the composite structure fed to a cutting station where strips are cut or stamped transversely to machine direction of the web to obtain individual adhesive bandages. As the bandages are cut from the composite web, the edges of the pad area are heat-sealed along a narrow band to form a well-defined pad area which totally encloses the deposited material. The resulting bandages are ready to be packaged and sterilized.

Island bandages according to the present invention are prepared by depositing the medicated gel onto the center of the base fabric in spots spaced at intervals in registry with the desired pad area of the final bandage. The pad fabric, preferably of the same width as the base fabric, is applied and the combined fabrics passed through an embossing station where the fabric surrounding the pad area is compacted and heat-fused to form a central blister pad surrounded by a compacted, non-woven sheet-like structure.

The adhesive is applied to the compacted portion of the bandage fabric by' printing or other suitable means. Adhesive release liners are applied over the adhesive coated areas and the bandages die cut to forn individual island bandages having a central blister pad area. The resulting bandages are packaged and sterilized using conventional procedures.

The material used in the fabrication of bandages according to the present invention is preferably a nonwoven fabric composed of absorbent fibers such as cellulose or rayon and heat-fusible fibers such as polyethylene or polypropylene, in relative proportions such that the pad of the finished bandage is soft and absorbent while the heat bonded and compacted areas are strong and stable.

FIGURE 1 is schematic representation in perspective of a process used to produce strip adhesive bandages of the present invention.

FIGURE 2 is a cross-sectional view in perspective of the base web of FIGURE 1 through line 2-2.

FIGURE 3 is a cross-sectional view in perspective of the base web after application of the pad, through line 3-3 of FIGURE 1.

FIGURE 4 is a cross-sectional view in perspective of the base web and pad after application of adhesive, through line 4-4 of Figure 1.

FIGURE 5 is a cross-sectional view in perspective of the base web and pad of FIGURE 4 after application of adhesive release papers, through line 5-5 of FIGURE 1.

FIGURE 6 is a cross-sectional view in perspective of the base web and pad of FIGURE 5 after cutting into strips, through line 6-6 of FIGURE 1.

FIGURE 7 is a top plan view from the pad side of a strip adhesive bandage according to the present invention, with the adhesive release strips removed.

FIGURE 8 is a top plan view from the pad side of a square island adhesive bandage according to the present invention, with the adhesive release strips removed.

FIGURE 9 is a top plan view from the pad side of a spot adhesive bandage according to the present invention, with the adhesive release strips removed.

The strip adhesive bandages of the present invention are fabricated from continuous lengths of bandage material which are preferably bulky, heat-fusible, absorbent, nonwoven fabrics. The bandage is fabricated by positioning the pad material over the center area of the base fabric, securing the pad by heat welding the edges thereof, and heat fusing the edge portions of the base fabric extending from the pad area to provide surfaces capable of accepting an adhesive. After applying the adhesive and covering the adhesive surfaces with release liners, the composite web is cut transversely to its machine direction in strips the width of the desired bandage.

The central pad portion of the bandage comprises a double thickness of the starting bandage materials. Since it is generally desirable for the bandage pad to be absorbent as well as providing a cushioning effect, it is important for the initial bandage materials to also possess these properties. In addition, since the fabric forming the pad must be secured in some manner, the bandage materials are preferably a heat-fusible composition which permits the pad fabric to be continuously and permanently heat-welded along the edges to the base fabric.

A bandage material meeting all the above requirements is a nonwoven fabric comprising a mixture of cellulose or other absorbent fibers and polyethylene or other heat-fusible fibers. The heat-fusible fibers are interspersed throughout the web and are preferably present in an amount of at least 10% by weight. The fabric preferably has sufficient thickness or bulk so that the triple-layered pad has a thickness of at least 2 mm in the final bandage. Nonwoven webs useful in the practice of the present invention are known in the art for use in other applications. See, for example, U.S. Patents Nos. 2,774,128; 3,067,747; 4,083,913; 4,160,159; and 4,307,721.

A particularly preferred bandage material is a low density, highly absorbent, thermal bonded nonwoven fabric comprising absorbent fibers and staple length polyesterpolyethylene conjugate fibers. These nonwoven fabrics are produced by a process which includes producing a web comprising absorbent fibers and staple length polyester/polyethylene conjugate fibers; subjecting the web to a temperature sufficient to fuse the lower melting component of the conjugate fibers without facing the higher melting component while maintaining the web under little or no compression; and cooling the web to resolidify the lower melting component of the conjugate fibers, thereby forming a nonwoven fabric bonded at sites where the conjugate fibers touch each other and adjacent absorbent fibers.

A particularly preferred nonwoven fabric is a laminate comprising a core of a mixture of short-length natural cellulose fibers and staple length polyester/polyethylene conjugate fibers, and a light weight veneer of heat fusible fibers on each surface of the core. The composite web is passed through a through-air heater to fuse the lower melting component of the conjugate fibers while maintaining the fibrous integrity of these fibers, and to fuse or soften the surfaces of the heat-fusible fibers in the two outer veneers. As the material emerges from the heater and cools, the fused surfaces of the lower melting component of the conjugate fibers, i.e., the polyethylene, solidify, and bonds form where these surfaces touch each other and other fibers.

The thermal-bonded, nonwoven fabrics particularly useful in the practice of the present invention employ polyester/ polyethylene conjugate fibers wherein at least about 50 percent of the surface of the individual fibers is polyethylene. Most preferred are sheath/core fibers with the polyethylene as the sheath and the polyester as the core. The fibers will usually have a denier within the range of from about 1 to about 6, and a length within the range of from about 1/2 inch to about 3 or 4 inches.

Absorbent fibers employed in such thermal-bonded nonwoven fabrics include rayon staple fibers, cotton fibers, short length natural cellulose fibers such as wood pulp fibers and cotton linters, and mixtures thereof.

Heat-fusible fibers used in the veneer of the nonwoven fabric are preferably staple length conjugate fibers. However, if desired, other types of heat-fusible fibers such as polypropylene homofil fibers can be used in the veneer. The veneer can also contain other fibers, such as rayon, cotton, or polyester staple fibers.

The above bonded, nonwoven fabrics normally have basis weights from about 1/2 to about 6 ounces per square yard. The bulk density of the fabrics is usually below about 0.15 gram per cubic centimeter, preferably below about 0.09 gram per cubic centimeter, e.g., from about 0.02 to about 0.09 gram per cubic centimeter, and more preferably, from about 0.025 to about 0.06 gram per cubic centimeter. The fabrics preferably have an absorbent capacity, as measured by a Gravimetric Absorbency Tester, of at least 600 percent and preferably at least 1400 percent, exclusive of any nonabsorbent layer such as a veneer of 100 percent fusible fibers.

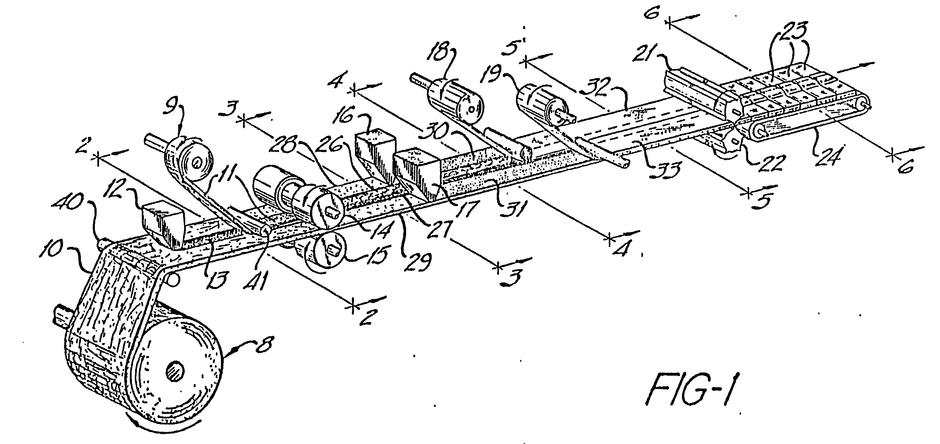

The process of preparing adhesive strip bandages from continuous rolls of bonded, nonwoven fabric bandage material will be better understood by reference to FIGURES 1 through 6.

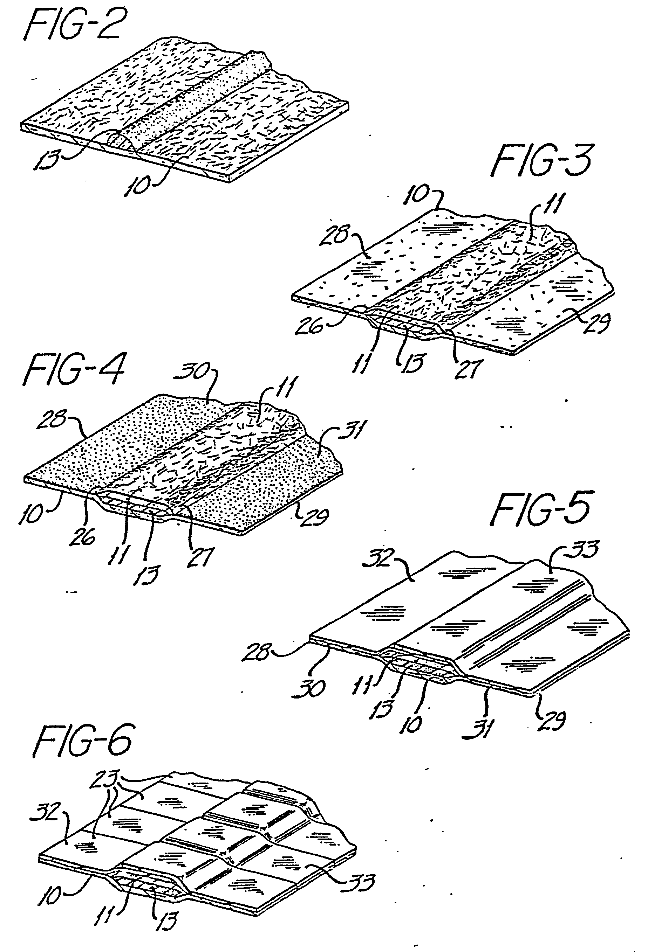

In FIGURE 1, the center portion of nonwoven base fabric 10 from roll 8 passes over idler roll 40 and under metering apparatus 12 where a bead or spots of medicated gel or other material 13 is deposited onto the center line of the fabric. The cross section of the base fabric carrying the gel is illustrated in FIGURE 2. Nonwoven pad fabric 11 feeding from roll 9 passes under idler roll 41 which is adjusted to provide clearance for the gel between the pad and base fabrics. The composite fabric next passes between hot calendaring rolls 14 and 15, which heat the material to about 100 to 150°C, where the edges 26 and 27 of the pad fabric are welded to the base fabric and side widths 28 and 29 of fabric extending from the pad area are compacted under heat and pressure to form a thin, dense, sheet-like material. Roll 14 includes a bridging center to avoid compacting the pad area of the fabric. The cross section or the resulting product is illustrated in FIGURE 3.

The composite fabric next passes through the adhesive application station where a pressure-sensitive, skin-compatible adhesive is applied from reservoirs 16 and 17 to the compacted side portions of the material, the adhesive coating being designated as 30 and 31. FIGURE 4 is a view in cross section of the adhesive-coated composite fabric. The adhesive may be any pressure-sensitive, medical grade adhesive suitable for use in adhesive bandages, and is preferably a hypoallergenic hot melt adhesive. Emulsion adhesives may also be used provided the adhesive application station includes means for drying the adhesive after application.

As the adhesive-coated material continues through the process, the adhesive coating and the central pad area are covered by release papers 32 and 33 fed from rolls 18 and 19 respectively. The release papers preferably overlap along the center of the web over the pad area. FIGURE 5 is a view in cross section of the composite material after application of the release papers.

The composite material next passes through a cutting station where cutters 21 and 22 cut the material into transverse strips 23 while simultaneously heat-fusing the cut edges of the pad fabric to enclose the gel material. The cut strips are carried on conveyor belt 24 to a packaging station (not shown) where individual strips are packaged in sealed envelopes prior to sterilization. The cut strips 23 are further illustrated in cross section in FIGURE 6.

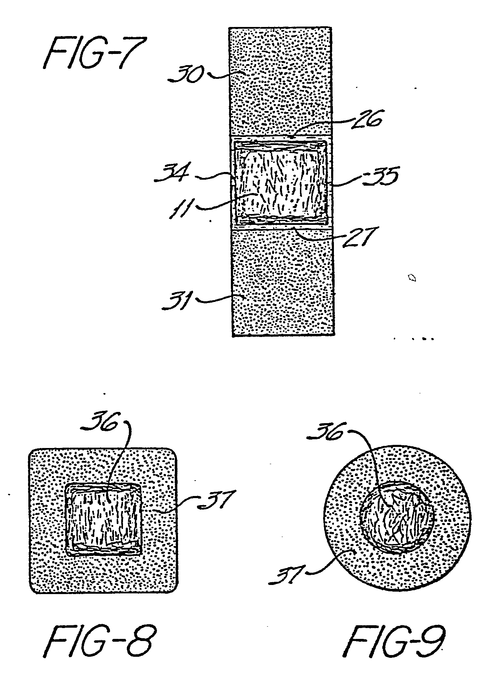

An individual strip bandage produced according to the present invention is illustrated in FIGURE 7 with the adhesive release papers removed. In the illustrated bandage, pad 25 is set in from the longitudinal edges of the bandage by heat-fused areas 34 and 35 to form a well defined pad portion which totally encloses the medicated gel or other material contained between the base fabric and the pad fabric.

Other bandage configurations and constructions utilizing the inventive concepts of the present invention will be apparent to those skilled in the art, the principle feature of the present invention being the construction of an adhesive bandage and pad utilizing heat-fusible bandage materials, preferably continuous webs comprising heat fusible, fibers.

Representative of such other bandage configurations are the square island bandage illustrated in FIGURE 8 and the spot bandage illustrated in FIGURE 9, both of which are herein referred to as island bandages. Island bandages are characterized by centrally located blister pad area 36 surrounded by an adhesive-coated area 37. To provide an adhesive area of uniform thickness throughout, it is desirable for the pad fabric used in the manufacture of the bandage to extend over the entire width of bandage rather than simply over the pad area as in the case of the strip bandage. Additionally, to prevent the medicated gel from interfering with compaction of the fabric and application of the adhesive, the gel is applied to the base fabric in spots in registry with the final pad area of the bandages.

The pad area of the island bandage is sealed by passing the fabrics through a hot embossing station, with rollers configured according to the size and shape of the desired bandage whereby the areas of the bandage surrounding the pad are heat-compacted to form a dense, sheet-like, structure capable of accepting an adhesive coating. The adhesive is preferably applied by transfer coating or other convenient method to provide a continuous coating of adhesive over the compacted material surrounding the pad area. Adhesive release liners are applied in continuous strips overlapping the central pad area, and individual bandages then die cut from the continuous feed of composite material.

Bandage flexibility and bias characteristics can be modified by altering fiber type and orientation. The bandage material can be further modified by incorporating fiber finishes to vary absorbency characteristics if desired. The surface of the pad intended for placement against the wound may be heat glazed or otherwise surface modified to provide wound release characteristics without significantly affecting the bulk or absorbency of the pad. These and other variations which will be apparent to those skilled in the art are included within the scope of the present invention as defined by the claims.

## Claims

1. An adhesive bandage constructed of:
a heat fusible base portion (10); and
a bulky, heat fusible pad portion (11) centrally located on said base portion (10);
said pad portion (11) having been provided separate from said base portion (10) and being heat fused to said base portion (10) along its perimeter;
said base portion (10) including adhesive-coated areas (28, 29) extending outwardly from said pad portion (11),
characterised in that:
said pad portion (11) is formed of a non-woven fabric and is heat fused to said base portion (10) around its entire perimeter, whereby the interior of said pad portion (11) is totally enclosed and forms a blister pad on said base portion (10); and

said adhesive coated areas (28, 29) of said base portion (10) have been compacted and heat fused from a bulky non-woven fabric into a dense, sheet-like structure.

2. The bandage of Claim 1, wherein a medicinal or therapeutic composition (13) is enclosed within the confines of the pad portion (11).

3. The bandage of Claim 1 or Claim 2, wherein the outer surface of said pad portion (11) is characterized by having wound release properties.

4. The bandage of any one of Claims 1 to 3, wherein said non-woven fabric comprises a mixture of absorbent fibers with at least 10% heat fusible fibers.

5. The bandage of Claim 4, wherein said heat fusible fibers are staple length polyester core/polyethylene sheath conjugate fibers.

6. The bandage of any one of Claims 1 to 3, wherein said non-woven fabric comprises a core of a mixture of absorbent fibers and heat fusible polyester/polyethylene conjugate fibers, and an outer veneer on both faces of said core comprising a non-woven web of heat fusible polyester/polyethylene conjugate fibers.

7. The bandage of Claim 6, wherein the surface of said pad comprising a veneer of said heat fusible fibers is heat-glazed to impart nonsticking, wound release properties to said pad.

8. The bandage of any one of Claims 4 to 7, wherein said absorbent fibers are rayon, cotton or wood pulp fibers, cotton linters, or a mixture thereof.

9. The bandage of any one of Claims 1 to 8, wherein the adhesive is a pressure sensitive adhesive.

10. The bandage of any one of Claims 1 to 9, wherein the base portion (10) is elongated and rectangular and the pad portion (11) is substantially square, whereby the bandage is a strip bandage.

11. The bandage of any one of Claims 1 to 9, in the form of an island bandage.

12. A method, for the continuous production of a plurality of adhesive bandage strips (23) from a heat-fusible material, comprising the steps of:

a) providing a continuous source (8) of base material (10) having a width corresponding to the desired length of the bandage strip (23);

b) providing a continuous source (9) of pad material having a width at least equal to the desired length of the pad (11);

c) centering the pad material (11) on the base material (10);

d) securing the pad material (11) on the base material (10) by heat fusing the edges of the pad material (11) to the base material (10);

e) applying an adhesive coating (30, 31) to one surface of the side portions (28, 29) of said base material, (10) extending from the pad material;

f) covering said adhesive-coated side portions (28, 29) of said base material with a release liner; and

g) cutting the resulting composite material into narrow strips (23) transversely to the longitudinal machine direction thereof,

characterised in that:

said base material (10) comprises a bulky, non-woven fabric;

said pad material comprises a separate bulky, non-woven fabric;

during said securing step d), the edges of the pad material (11) are compacted;

prior to said adhesive applying step e), said side portions (28, 29) of the base material (10) are compacted and heat fused to obtain a dense, sheet-like structure for receiving the adhesive; and

during the cutting step g), the cut edges of the pad material (11) are heat fused to seal the pad material (11) to the base material (10), whereby there are formed individual adhesive bandages comprising a central enclosed blister pad portion and adhesive-coated side portions extending therefrom.

13. A method, for the continuous production of a plurality of adhesive island bandages from a heat-fusible material, comprising the steps of:

a) providing a continuous source of base material (37) having a width corresponding to the desired width of the bandage;

b) providing a continuous source of pad material (36) having a width at least equal to the desired width of the pad;

c) centering the pad material (36) on the base material (37);

d) securing the pad material (36) to the base material (37) by heat-fusing the pad material (36) to the base material (37);

e) applying an adhesive coating to one surface of the heat-fused portions of said pad material (36) and the portions of the base material (37) extending beyond the pad area;

f) covering said adhesive-coated portions of said material with a release liner; and

g) cutting bandages from the resulting composite material

characterised in that:

said base material (37) comprises a bulky, non-woven fabric;

said pad material (36) comprises a separate bulky, non-woven fabric;

during said securing step d), the pad material (36) is heat-fused to the base material (37) all around the pad material perimeter and the perimeter of the pad material is compacted;

prior to said adhesive applying step e), the portions of the base material (37) extending beyond the pad area are compacted and heat fused to obtain a dense, sheet-like structure for receiving the adhesive;

and during the cutting step g), there are formed individual adhesive island bandages comprising a central enclosed blister pad portion surrounded by adhesive-coated side portions extending therefrom.

14. The method of Claim 12 or Claim 13, wherein a medicinal or therapeutic composition (13) is enclosed within the confines of the pad portion (11).

15. The method of any one of Claims 12 to 14, wherein said non-woven fabric comprises a mixture of absorbent fibers with at least 10% heat fusible fibers.

16. The method of Claim 15, wherein said heat fusible fibers are staple length polyester core/polyethylene sheath conjugate fibers.

17. The method of any one of Claims 12 to 14, wherein said non-woven fabric comprises a core of a mixture of absorbent fibers and heat fusible polyester/polyethylene conjugate fibers, and an outer veneer on both faces of said core comprising a nonwoven web of heat fusible polyester/polyethylene conjugate fibers.

18. The method of claim 17, wherein the surface of said pad, comprising a veneer of said heat fusible fibers, is heat glazed to impart nonsticking, wound release properties to said pad.

19. The method of any one of claims 13 to 18, wherein said absorbent fibers are rayon, cotton or wood pulp fibers, cotton linters, or a mixture thereof.

20. The method of any one of claims 11 to 19, wherein said edges of said pad material are secured to said base material and said side portions of said base material are compacted and heat fused by compressing said bulky, non-woven material while heated to 100 to 150°C.

## Patentansprüche

1. Klebeverband, welcher aufgebaut ist aus:
einem warmverschweißbaren Unterlagsteil (10); und einem bauschigen, warmverschweißbaren Kissenteil (11), der auf dem genannten Unterlagsteil (10) zentral angeordnet ist;
wobei der genannte Kissenteil (11) getrennt von dem genannten Unterlagsteil (10)bereitgestellt und mit dem genannten Unterlagsteil (10) längs des Umfanges des Kissenteils warmverschweißt worden ist;
und wobei der genannte Unterlagsteil (10) mit Klebstoff überzogene Flächenteile (28,29) aufweist, die sich von dem genannten Kissenteil (11) nach außen erstrecken;
dadurch gekennzeichnet, daß der genannte Kissenteil (11) aus einem Faservliesstoff gebildet und mit dem genannten Unterlagsteil (10) längs des gesamten Umfanges des Kissenteils warmverschweißt ist, wodurch das Innere des genannten Kissenteiles (11) zur Gänze umschlossen ist und auf dem genannten Unterlagsteil (10) ein Wundkissen bildet und
daß die genannten, mit Klebstoff überzogenen Flächenteile (28, 29) des genannten Unterlagsteiles (10) aus einem bauschigen Faservliesstoff zu einer dichten, blattartigen Struktur verdichtet und warmverschweißt worden sind.

2. Verband nach Anspruch 1, worin eine medizinische oder therapeutische Zusammensetzung (13) innerhalb der Begrenzungen des Kissenteiles (11) eingeschlossen ist.

3. Verband nach Anspruch 1 oder Anspruch 2. wobei die Außenseite des genannten Kissenteiles (11) dadurch gekennzeichnet ist, daß sie die Eigenschaft der leichten Abziehbarkeit von einer Wunde aufweist.

4. Verband nach einem der Ansprüche 1 bis 3, worin der genannte Faservliesstoff ein Gemisch aus absorbierenden Fasern mit wenigstens 10 % warmverschweißbaren Fasern enthält.

5. Verband nach Anspruch 4, wobei die genannten warmverschweißbaren Fasern Konjugatfasern mit Stapelfaserlänge sind, die aus einem Polyesterkern und einer Polyethylenschale bestehen.

6. Verband nach einem der Ansprüche 1 bis 3, wobei der genannte Faservliesstoff einen Kern aus einem Gemisch aus absorbierenden Fasern und aus warmverschweißbaren Polyester/Polyethylen-Konjugatfasern, sowie eine äußere Deckschicht auf beiden Seiten des genannten Kerns aufweist, welche Deckschicht eine Faservliesstoffbahn aus warmverschweißbaren Polyester/Polyethylen-Konjugatfasern umfaßt.

7. Verband nach Anspruch 6, wobei die Oberfläche des genannten Kissens, welches eine Deckschicht aus den genannten, warmverschweißbaren Fasern umfaßt, unter Warmeeinwirkung geglättet worden ist, um dem genannten Kissen Eigenschaften solcher Art zu verleihen, daß es nicht klebend ist und sich von einer Wunde leicht abziehen läßt.

8. Verband nach einem der Ansprüche 4 bis 7, wobei die genannten absorbierenden Fasern Reyon-, Baumwoll- oder Holzzellstoffasern, Baumwoll-Linters oder ein Gemisch davon sind.

9. Verband nach einem der Ansprüche 1 bis 8, wobei der Klebstoff ein Haftklebstoff ist.

10. Verband nach einem der Ansprüche 1 bis 9, wobei der Unterlagsteil (10) langgestreckt und rechteckig ist, und wobei der Kissenteil (11) im wesentlichen quadratisch ist, wodurch der Verband ein Streifenverband ist.

11. Verband nach einem der Ansprüche 1 bis 9, in der Form eines Inselverbandes.

12. Verfahren zur kontinuierlichen Erzeugung einer Mehrzahl von Klebeverbandsstreifen (23) aus einem warmverschweißbaren Material, welches Verfahren die folgenden Stufen umfaßt:
a) das Bereitstellen einer kontinuierlichen Quelle (8) von Unterlagsmaterial (10) mit einer Breite, welche der gewünschten Länge des Verbandstreifens (23) entspricht;
b) das Bereitstellen einer kontinuierlichen Quelle (9) von Kissenmaterial mit einer Breite, die wenigstens gleich groß wie die gewünschte Länge des Kissens (11) ist;
c) das Zentrieren des Kissenmaterials (11) auf dem Unterlagsmaterial (10).

d) das Befestigen des Kissenmaterials (11) auf dem Unterlagsmaterial (10) durch Warmverschweißen der Ränder des Kissenmaterials (11) mit dem Unterlagsmaterial (10);

e) das Auftragen eines Klebstoffüberzuges (30, 31) auf eine Oberfläche der Seitenteile (28, 29) des genannten Unterlagsmaterials (10), welche Seitenteile sich von dem Kissenmaterial weg erstrecken;

f) das Bedecken der genannten, mit Klebstoff überzogenen Seitenteile (28, 29) des genannten Unterlagsmaterials mit einem Abziehstreifen; und

g) das Zerschneiden des so erhaltenen Verbundmaterials zu schmalen Streifen (23), und zwar quer zur in der Längsrichtung verlaufenden Laufrichtung des Verbundmaterials;

dadurch gekennzeichnet, daß:

das genannte Unterlagsmaterial (10) einen bauschigen Faservliesstoff umfaßt;

das genannte Kissenmaterial einen getrennten, bauschigen Faservliesstoff umfaßt;

während der genannten Stufe d) des Befestigens, die Ränder des Kissenmaterials (11) verdichtet werden;

vor der genannten Stufe e) des Klebstoffauftrages die genannten Seitenteile (28,29) des Unterlagsmaterials (10) verdichtet und warmverschweißt werden, um eine dichte, blattartige Struktur für die Aufnahme des Klebstoffes zu erzielen; und

daß während der Stufe g) des Schneidens die geschnittenen Ränder des Kissenmaterials (11) warmverschweißt werden, um das Kissenmaterial (11) mit dem Unterlagsmaterial (10) zu versiegeln, wodurch einzelne Klebeverbände gebildet werden, welche einen zentralen, umschlossenen Wundkissenteil und sich davon weg erstreckende, mit Klebstoff überzogene Seitenteile umfassen.

13. Verfahren zur kontinuierlichen Erzeugung einer Mehrzahl von Inselklebeverbänden aus einem warmverschweißbaren Material, welches Verfahren die folgenden Stufen umfaßt:

a) das Bereitstellen einer kontinuierlichen Quelle von Unterlags material (37) mit einer Breite, welche der gewünschten Breite des Verbandes entspricht;

b) das Bereitstellen einer kontinuierlichen Quelle von Kissenmaterial (36) mit einer Breite, die wenigstens gleich groß wie die gewünschte Breite des Kissens ist;

c) das Zentrieren des Kissenmaterials (36) auf dem Unterlagsmaterial (37).

d) das Befestigen des Kissenmaterials (36) auf dem Unterlagsmaterial (37) durch Warmverschweißen des Kissenmaterials (36) mit dem Unterlagsmaterial (37);

e) das Auftragen eines Klebstoffüberzuges auf eine Oberfläche der warmverschweißten Teile des genannten Kissenmaterials (36) und auf diejenigen Teile des Unterlagsmaterials (37), die sich über den Kissenbereich hinaus erstrecken;

f) das Bedecken der genannten, mit Klebstoff überzogenen Teile des genannten Materials mit einem Abziehstreifen; und

g) das Schneiden von Verbänden aus dem entstandenen Verbundmaterial;

dadurch gekennzeichnet, daß:

das genannte Unterlagsmaterial (37) einen bauschigen Faservliesstoff umfaßt,

das genannte Kissenmaterial (36) einen getrennten, bauschigen Faservliesstoff umfaßt;

während der genannten Stufe d) des Befestigens, das Kissenmaterial (36) rund um den gesamten Umfang des Kissenmaterials herum mit dem.

Unterlagsmaterial (37) warmverschweißt wird, und der Umfang des Kissenmaterials verdichtet wird;

vor der genannten Stufe e) des Klebstoffauftrages, diejenigen Teile des Unterlagsmaterials (37), die sich über den Kissenbereich hinaus erstrecken, verdichtet und warmverschweißt werden, um eine dichte, blattartige Struktur für die Aufnahme des Klebstoffes zu erzielen; und daß während der Stufe g) des Schneidens einzelne Inselklebeverbände gebildet werden, welche einen zentralen, umschlossenen Wundkissenteil aufweisen, der von sich davon weg erstreckenden, mit Klebstoff überzogenen Seitenteilen umgeben ist.

14. Verfahren nach Anspruch 12 oder Anspruch 13, worin eine medizinische oder therapeutische Zusammensetzung (13) innerhalb der Begrenzungen des Kissenteiles (11) eingeschlossen ist.

15. Verfahren nach einem der Ansprüche 12 bis 14, worin der genannte Faservliesstoff ein Gemisch aus absorbierenden Fasern mit wenigstens 10 % warmverschweißbaren Fasern enthält.

16. Verfahren nach Anspruch 15, wobei die genannten, warmverschweißbaren Fasern Konjugatfasern mit Stapelfaserlänge sind, die aus einem Polyesterkern und einer polyethylenschale bestehen.

17. Verfahren nach einem der Ansprüche 12 bis 14, wobei der genannte Faservliesstoff einen Kern aus einem Gemisch aus absorbierenden Fasern und aus warmverschweißbaren Polyester/Polyethylen-Konjugatfasern, sowie eine äußere Deckschicht auf beiden Seiten des genannten Kerns aufweist, welche Deckschicht eine Faservliesstoffbahn aus warmverschweißbaren Polyester/Polyethylen-Konjugatfasern umfaßt.

18. Verfahren nach Anspruch 17, wobei die Oberfläche des genannten Kissens, welches eine Deckschicht aus den genannten, warmverschweißbaren Fasern umfaßt, unter Warmeinwirkung geglättet worden ist, um dem genannten Kissen Eigenschaften solcher Art zu verleihen, daß es nicht klebend ist und sich von einer Wunde leicht abziehen läßt.

19. Verfahren nach einem der Ansprüche 13 bis 18, wobei die genannten absorbierenden Fasern Reyon-, Baumwoll- oder Holzzellstoffasern, Baumwoll-Linters oder ein Gemisch davon sind.

20. Verfahren nach einem der Ansprüche 11 bis 19, wobei die genannten Ränderdes genannten Kissenmaterials an dem genannten Unterlagsmaterial dadurch befestigt werden, und die genannten Seitenteile des genannten Unterlagsmaterials dadurch verdichtet und warmverschweißt werden, daß das genannte bauschige Faservliesstoffmaterial komprimiert wird, während es auf 100 bis 150°C erhitzt wird.

**Revendications**

1. Pansement adhésif constitué:
d'une partie de base fusible à chaud (10), et
d'une partie de tampon rendue volumineuse, fusible à chaud (11), centrée sur la partie de base (10).
la partie de tampon (11) étant fournie séparée de la partie de base (10) et étant soudée à chaud à cette partie de base (10) le long de son périmètre;
la partie de base (10) comprenant des zones revêtues d'adhésif (28, 29) qui s'étendent vers l'extérieur à partir de la partie de tampon (11),
caractérisé en ce que la partie de tampon (11) est faite d'un tissu non tissé et est soudée à chaud à la partie de base (10) tout autour de son périmètre, l'intérieur de la partie de tampon (11) étant ainsi entièrement clos et formant un tampon-cloque sur la partie de base (10), et les zones revêtues d'adhésif (28, 29) de la partie de base (10) ayant été comprimées et soudées à chaud, ce qui transforme un tissu non tissé volumineux en une structure dense en forme de feuille.

2. Pansement suivant la revendication 1, dans lequel une composition médicinale ou thérapeutique (13) est enfermée dans les confins de la partie de tampon (11).

3. Pansement suivant la revendication 1 ou 2, dans lequel la surface extérieure de la partie de tampon (11) est caractérisée en ce qu'elle possède des propriétés antiadhésives à l'égard d'une plaie.

4. Pansement suivant l'une quelconque des revendications 1 à 3, dans lequel le tissu non tissé comprend un mélange de fibres absorbantes et d'au moins 10% de fibres fusibles à chaud.

5. Pansement suivant la revendication 4, dans lequel les fibres fusibles à chaud sont des fibres conjuguées en longueurs coupées à âme de polyester et gaine de polyéthylène.

6. Pansement suivant l'une quelconque des revendications 1 à 3, dans lequel le tissu non tissé comporte une âme faite d'un mélange de fibres absorbantes et de fibres conjuguées fusibles à chaud de polyester/polyéthylène, ainsi qu'un parement extérieur, sur les deux faces de l'âme, comprenant une nappe non tissée de fibres conjuguées fusibles à chaud de polyester/polyéthylène.

7. Pansement suivant la revendication 6, dans lequel la surface du tampon comprenant un parement des fibres fusibles à chaud est glacée à chaud pour conférer au tampon un caractère non poisseux qui le rend non adhérent à l'égard d'une plaie.

8. Pansement suivant l'une quelconque des revendications 4 à 7, dans lequel les fibres absorbantes sont des fibres de rayonne, de coton ou de pâte de bois, de bourre de coton ou un mélange de ces fibres.

9. Pansement suivant l'une quelconque des revendications 1 à 8, dans lequel l'adhésif est un adhésif sensible à la pression.

10. Pansement suivant l'une quelconque des revendications 1 à 9, dans lequel la partie de base (10) est allongée et rectangulaire et la partie de tampon (11) est en substance carrée, le pansement étant un pansement en bande.

11. Pansement suivant l'une quelconque des revendications 1 à 9 ayant la forme d'un pansement à îlot.

12. Procédé pour la fabrication en continu de plusieurs bandes de pansements adhésifs (23) à partir d'une matière fusible à chaud, comprenant les opérations suivantes:
a) fournir une source continue (8) de matière de base (10) ayant une largeur correspondant à la longueur souhaitée de la bande de pansement (23);
b) fournir une source continue (9) de matière de tampon ayant une largeur au moins égale à la longueur souhaitée du tampon (11);
c) centrer la matière de tampon (11) sur la matière de base (10);
d) fixer la matière de tampon (11) sur la matière de base (10) par soudage à chaud des bords de la matière de tampon (11) à la matière de base (10);
e) appliquer un revêtement adhésif (30, 31) sur une surface des parties latérales (28, 29) de la matière de base (10) qui partent de la matière de tampon;
f) couvrir les parties latérales revêtues d'adhésif (28, 29) de la matière de base au moyen d'une garde antiadhésive, et
g) couper la matière composite résultante en des bandes étroites (23) transversalement à leur sens machine longitudinal,
caractérisé en ce que:
la matière de base (10) comprend un tissu non tissé rendu volumineux;
la matière de tampon comprend un tissu non tissé rendu volumineux, séparé;
pendant l'opération de fixation d), les bords de la matière de tampon (11) sont comprimés;
avant l'opération d'application de l'adhésif e), les Parties latérales (28, 29) de la matière de base (10) sont comprimées et soudées à chaud pour obtenir une structure dense en forme de feuille destinée à recevoir l'adhésif, et
pendant l'opération de coupe g), les bords coupés de la matière de tampon (11) sont soudés à chaud pour sceller la matière de tampon (11) à la matière de base (10), de manière à former des pansements adhésifs individuels comprenant une partie centrale close formant tampon-cloque et des parties latérales revêtues d'adhésif qui partent de cette partie centrale.

13. Procédé pour la fabrication en continu de plusieurs pansements à îlots adhésifs à partir d'une matière fusible à chaud, comprenant les opérations suivantes:

a) fournir une source continue de matière de base (37) ayant une largeur correspondant à la largeur souhaitée du pansement;

b) fournir une source continue de matière de tampon (36) ayant une largeur au moins égale à la largeur souhaitée du tampon;

c) centrer la matière de tampon (36) sur la matière de base (37);

d) fixer la matière de tampon (36) à la matière de base (37) par soudage à chaud de cette matière de tampon (36) à la matière de base (37);

e) appliquer un revêtement adhésif sur une surface des parties soudées à chaud de la matière de tampon (36) et sur les parties de la matière de base (37) qui s'étendent au-delà de la zone de tampon;

f) couvrir les parties revêtues d'adhésif de la matière au moyen d'une garde antiadhésive, et

g) couper des pansements à partir de la matière composite obtenue,

caractérisé en ce que:

la matière de base (37) comprend un tissu non tissé rendu volumineux;

la matière de tampon (36) comprend un tissu non tissé rendu volumineux, séparé;

pendant l'opération de fixation d), la matière de tampon (36) est soudée à chaud tout autour de son périmètre à la matière de base (37), et le périmètre de cette matière de tampon est comprimé;

avant l'opération d'application d'adhésif e), les parties de la matière de base (37) qui s'étendent au-delà de la zone du tampon sont comprimées et soudées à chaud pour obtenir une structure dense en forme de feuille destinée à recevoir l'adhésif, et

pendant l'opération de coupe g), des pansements à îlots adhésifs individuels sont formés et comprennent une partie centrale close formant tampon-cloque entourée par des parties latérales revêtues d'adhésif qui partent de la partie centrale.

14. Procédé suivant la revendication 12 ou 13, dans lequel une composition médicinale ou thérapeutique (13) est enfermée dans les confins de la partie de tampon (11).

15. Procédé suivant l'une quelconque des revendications 12 à 14, dans lequel le tissu non tissé comprend un mélange de fibres absorbantes et d'au moins 10% de fibres fusibles à chaud.

16. Procédé suivant la revendication 15, dans lequel les fibres fusibles à chaud sont des fibres conjuguées en longueurs coupées à âme de polyester et gaine de polyéthylène.

17. Procédé suivant l'une quelconque des revendications 12 à 14, dans lequel le tissu non tissé comprend une âme faite d'un mélange de fibres absorbantes et de fibres conjuguées fusibles à chaud de polyester/polyéthylène, ainsi qu'un parement extérieur sur les deux faces de l'âme, comprenant une nappe non tissée de fibres conjuguées fusibles à chaud de polyester/polyéthylène.

18. Procédé suivant la revendication 17, dans lequel la surface du tampon, comprenant un parement des fibres fusibles à chaud, est glacée à chaud pour conférer au tampon un caractère non poisseux qui le rend non adhérent à l'égard d'une plaie.

19. Procédé suivant l'une quelconque des revendications 13 à 18, dans lequel les fibres absorbantes sont des fibres de rayonne, de coton ou de pâte de bois, de bourre de coton ou un mélange de telles fibres.

20. Procédé suivant l'une quelconque des revendications 11 à 19, dans lequel les bords de la matière de tampon sont fixés à la matière de base et les parties latérales de la matière de base sont comprimées et soudées à chaud par compression de la matière non tissée rendue volumineuse tandis qu'elle est chauffée à une température de 100 à 150°C.

FIG-1

EP 0 120 612 B1

FIG-2

FIG-3

FIG-4

FIG-5

FIG-6

EP 0 120 612 B1

FIG-7

30

26

34

35

11

27

31

FIG-8

36

31

FIG-9

36

31

3